(19) Europäisches Patentamt
European Patent Office
Office européen des brevets

(11) Numéro de publication: **0 317 426 B1**

(12) # FASCICULE DE BREVET EUROPEEN

(45) Date de publication de fascicule du brevet: **02.06.93**

(51) Int. Cl.[5]: **C07D 461/00**, A61K 31/435

(21) Numéro de dépôt: **88402871.3**

(22) Date de dépôt: **16.11.88**

Le dossier contient des informations techniques présentées postérieurement au dépôt de la demande et ne figurant pas dans le présent fascicule.

(54) **Produits optiquement actifs de dérivés de 20,21-dinoréburnaménine, leur procédé de préparation, leur application comme médicaments et les compositions pharmaceutiques les renfermant.**

(30) Priorité: **19.11.87 FR 8715979**

(43) Date de publication de la demande:
**24.05.89 Bulletin 89/21**

(45) Mention de la délivrance du brevet:
**02.06.93 Bulletin 93/22**

(84) Etats contractants désignés:
**AT BE CH DE ES FR GB GR IT LI LU NL SE**

(56) Documents cités:
**FR-A- 2 381 048**
**FR-A- 2 433 528**
**FR-A- 2 590 572**
**GB-A- 2 107 317**

**ARZNEIMITTEL-FORSCHUNG, vol. 36(II), no. 10, 1986, pages 1442-1448, Aulendorf, DE; F. BARZAGHI et al.: "A comparison of some of the pharmacological properties of the new eburnamenine derivative vindeburnol with those of vincamine, vinburnine, dihydroergotoxine mesilate and nicergoline"**

(73) Titulaire: **ROUSSEL-UCLAF**
**35, Boulevard des Invalides**
**F-75007 Paris(FR)**

(72) Inventeur: **Aktogu, Nurgün**
**2, rue Edmond About**
**F-92350 Le Plessis Robinson(FR)**
Inventeur: **Clémence, François**
**2, rue Turgot**
**F-75009 Paris(FR)**
Inventeur: **Oberlander, Claude**
**2, rue Paul Albert**
**F-75018 Paris(FR)**

(74) Mandataire: **Bourgouin, André et al**
**Département des Brevets ROUSSEL UCLAF**
**111, route de Noisy B.P. no 9**
**F-93230 Romainville (FR)**

EP 0 317 426 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

L'invention concerne les produits optiquement actifs de dérivés de la 20,21-dinoréburnaménine, leur procédé de préparation, leur application comme médicaments et les compositions pharmaceutiques les renfermant.

L'invention a pour objet les produits optiquement actifs des produits racémiques de formule (I) :

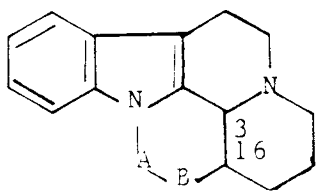

(I)

dans laquelle l'atome d'hydrogène en position 3 et l'atome d'hydrogène en position 16 sont trans et dans laquelle le groupement :

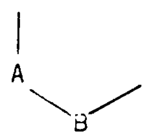

représente soit :

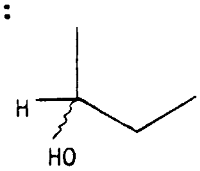

le radical hydroxy étant sous la forme alpha ou béta, soit :

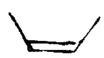

Les deux produits racémiques de formule (I) dans laquelle les atomes d'hydrogène en positions 3 et 16 sont trans et dans laquelle :

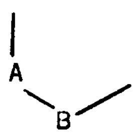

représente

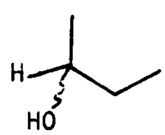

avec le radical hydroxyle dans l'une ou l'autre des deux orientations possibles sont décrits aux exemples 2 et 4 du brevet belge 864173 ou dans les brevets français 2 381 048 et 2 433 528.

Le produit racémique de formule (I) dans laquelle les atomes d'hydrogène en positions 3 et 16 sont trans et dans laquelle :

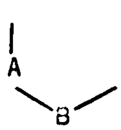

représente

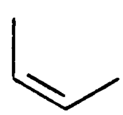

est décrit à l'exemple 1 du brevet français n¤ 2514357 ou britannique 2 107 317.

L'invention concerne notamment les produits optiquement actifs des produits racémiques de formula (I) dans laquelle le groupement :

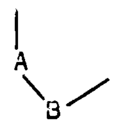 représente : 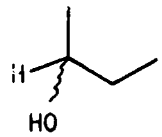 à savoir :

- le (14béta, 16alpha) 14,15-dihydro 20,21-dinoréburnamenin-14-ol,
- le (3alpha, 14béta) 14,15-dihydro 20,21-dinoréburnamenin-14-ol,
- le (14alpha, 16alpha) 14,15-dihydro 20,21-dinoréburnamenin-14-ol,
- et le (3alpha, 14alpha) 14,15-dihydro 20,21-dinoréburnamenin-14-ol

ainsi que leurs sels d'addition avec les acides minéraux ou organiques.

L'invention concerne aussi particulièrement les produits optiquement actifs des produits racémiques de formule (I) dans laquelle le groupement :

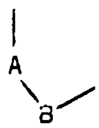 représente : 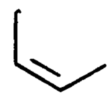 à savoir :

- la (16alpha) 20,21-dinoréburnaménine,
- et la (3alpha) 20,21-dinoréburnaménine,

ainsi que leurs sels d'addition avec les acides minéraux ou organiques.

Les sels d'addition avec les acides minéraux ou organiques des produits de formule (I) peuvent être, par exemple, les sels formés avec les acides chlorhydrique, bromhydrique, iodhydrique, nitrique, sulfurique, phosphorique, propionique, acétique, formique, benzoïque, maléïque, fumarique, succinique, tartrique, citrique, oxalique, glyoxylique, aspartique, ascorbique, les acides alcoylmonosulfoniques, tel que l'acide méthanesulfonique, l'acide éthanesulfonique, l'acide propanesulfonique, les acides alcoyldisulfoniques tels que l'acide méthanedisulfonique, l'acide alpha, béta-éthanedisulfonique, les acides arylmonosulfoniques, tels que l'acide benzènesulfonique et les acides aryldisulfoniques.

L'invention concerne aussi un procédé d'obtention des produits optiquement actifs, caractérisé en ce que :

a) on réduit soit le produit de formule (II) trans 3alpha, soit le produit de formule (II') trans 16alpha :

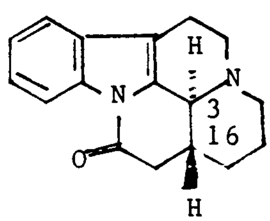 (II)

trans 3alpha

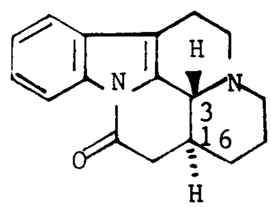 (II')

trans 16alpha

pour obtenir respectivement soit le produit de formule ($I_A$) trans 3alpha, soit le produit de formule ($I'_A$) trans 16alpha, produits de formules ($I_A$) et ($I'_A$) correspondant aux produits de formule (I) dans laquelle le groupement :

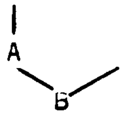 représente le groupement : 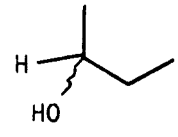

dans lequel le radical hydroxy est en position équatoriale :

b)on soumet ensuite, si désiré, soit le produit de formule ($I_A$) soit le produit de formule ($I'_A$) à l'action d'un acide pour obtenir soit le produit de formule ($I_{A1}$) trans 3alpha, soit le produit de formule ($I'_{A1}$) trans 16alpha, produits de formules ($I_{A1}$) et ($I'_{A1}$) correspondant aux produits de formule (I) dans laquelle le groupement :

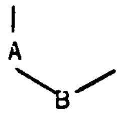

représente le groupement :

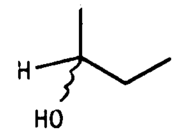

dans lequel le radical hydroxy est en position axiale :

c) on déshydrate ensuite, si désiré, soit l'un des deux produits de formules ($I_A$) ou ($I_{A1}$) soit l'un des deux produits de formules ($I'_A$) ou ($I'_{A1}$), pour obtenir soit le produit de formule ($I_C$) trans 3alpha, soit le produit de formule ($I'_C$) trans 16alpha, produits de formule ($I_C$) et ($I'_C$) correspondant aux produits de formule (I) dans laquelle le groupement :

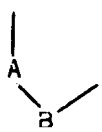

représente le groupement :

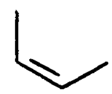

produits optiquement actifs que l'on soumet, si désiré, à l'action d'un acide minéral ou organique pour en former le sel.

Dans les conditions préférentielles de mise en oeuvre de l'invention, le procédé décrit ci-dessus est réalisé de la manière suivante :

- la réduction du produit de formule (II) ou du produit de formule (II') s'effectue avec un hydrure, tel que par exemple, l'hydrure mixte de lithium et d'aluminium, le diéthylhydrure de sodium et d'aluminium,
- l'acide utilisé pour obtenir le produit ($I_{A1}$) à partir du produit ($I_A$) ou le produit ($I'_{A1}$) à partir du produit ($I'_A$) est l'acide chlorhydrique.
- La séparation des produits ($I_{A1}$) et ($I_A$) et des produits ($I'_{A1}$) et ($I'_A$) s'effectue par chromatographie,
- l'agent de déshydratation utilisé pour obtenir les produits ($I_C$) et ($I'_C$) à partir des produits ($I_A$), ($I_{A1}$), ($I'_A$) ou ($I'_{A1}$) est un acide tel que l'acide chlorhydrique, l'acide bromhydrique, l'acide sulfurique, l'acide acétique, l'acide paratoluènesulfonique, l'acide méthanesulfonique.

Les produits optiquement actifs de l'invention ainsi que leurs sels d'addition avec les acides présentent d'intéressantes propriétés pharmacologiques.

Certains possèdent en particulier une affinité pour les récepteurs $alpha_2$ adrénergiques.

Les résultats donnés plus loin dans la partie expérimentale montrent une dissociation importante de l'affinité pour le récepteur $alpha_2$ entre les deux énantiomères de chaque produit racémique.

Les produits optiquement actifs de l'invention présentent aussi d'intéressantes propriétés anti-amnésiantes, anti-dépressives, protectrices neuronales, anti-anoxiques, anti-ischémiques, nootropes (effet anti-amnésiant dans un évitement passif et réversion d'un déficit mnésique après lésion cholinergique centrale).

Ces propriétés justifient leur application en thérapeutique et l'invention a également pour objet à titre de médicaments, les produits optiquement actifs tels que définis ci-dessus ainsi que leurs sels d'addition avec les acides minéraux ou organiques pharmaceutiquement acceptables.

L'invention a plus particulièrement pour objet, à titre de médicament :

- le (14alpha, 16alpha) 14,15-dihydro 20,21-dinoréburnamenin-14-ol,
- le (3alpha, 14béta) 14,15-dihydro 20,21-dinoréburnaménin-14-ol et leurs sels d'addition avec les acides pharmaceutiquement acceptables.

Les médicaments, objet de l'invention, peuvent être utilisés dans le traitement des insuffisances cérébrales d'origine anoxique et ischémique, dans les troubles de la mémoire, de l'attention et de la vigilance. Ils peuvent également être utilisés comme antidépresseurs.

L'invention s'étend aux compositions pharmaceutiques renfermant comme principe actif les médicaments définis ci-dessus.

Ces compositions pharmaceutiques peuvent être administrées par voie buccale, rectale, par voie parentérale ou par voie locale en application topique sur la peau et les muqueuses.

Ces compositions peuvent être solides ou liquides et se présenter sous les formes pharmaceutiques couramment utilisées en médecine humaine, comme, par exemple, les comprimés simples ou dragéifiés, les gélules, les granulés, les suppositoires, les préparations injectables, les pommades, les crèmes, les gels et les préparations en aérosols ; elles sont préparées selon les méthodes usuelles. Le principe actif peut y être incorporé à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

La posologie usuelle, variable selon le produit utilisé, le sujet traité et l'affection en cause, peut être, par exemple, de 10 mg à 200 mg par jour chez l'adulte, par voie orale.

Par ailleurs, les produits de formules II et II'utilisés comme produits de départ dans le procédé de l'invention, sont décrits dans le brevet belge n¤ 764166.

Les exemples donnés ci-après illustrent l'invention.

**Exemple 1 : (14béta, 16alpha) 14,15-dihydro 20,21-dinoréburnaménin-14-ol**.

On dissout 10,8 g de (16alpha) (+) 20,21-dinoréburnaménin-14(15H) one dans 110 cm3 de toluène anhydre, ajoute en dix minutes, sous atmosphère inerte, 18,9 cm3 de dihydrure de diéthyl aluminium-sodium à 25 % dans le toluène et agite une heure à température ampiante. On hydrolyse par addition de 20 cm3 de soude 5N et chauffe à 90¤C pendant deux heures. On distille le toluène et simultanément, on introduit 100 cm3 d'eau. On amène à température ambiante, essore le produit obtenu, lave à l'eau, sèche sous pression réduite et récupère 10,7 g de produit attendu qui recristallise dans le méthanol et fond à 254¤C. $[alpha]_D$ = -36¤ ± 1¤ (c = 0,6% DMF).

Dichroïsme circulaire (dioxanne)
Max. 225 nm $\Delta\epsilon$ = - 3
Max. 237 nm $\Delta\epsilon$ = + 9,5
Max. 280 nm $\Delta\epsilon$ = -2
Spectre RMN (pyridine) 250 MHZ ppm
Structure possible avec
OH équatorial
OH axial non détecté.

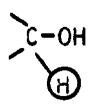 : 5,78 (m)

**Exemple 2 : (3alpha,14alpha) 14,15-dihydro 20,21-dinoréburnaménin-14-ol.**

On opère comme à l'exemple 1 à partir de 15 g de (3alpha) (-)(20,21-dinoréburnaménin-14(15H)-one et obtient 15 g de produit attendu contenant très peu de produit ayant l'OH axial. Après recristallisation dans le méthanol, on obtient le produit fondant à 254¤C.
$[alpha]_D$ = + 32,5¤ ± 1¤ (c = 1% DMF)

Dichroïsme circulaire (dioxanne)

Max. 227 nm $\Delta\epsilon$ = + 10
Max. 238 nm $\Delta\epsilon$ = - 10
Max. 288 nm $\Delta\epsilon$ = + 2

Spectre RMN 250 MHZ (pyridine) ppm

Structure possible avec OH équatorial
OH axial non détecté.

〉C-Ⓗ OH : 5,79 (m)

**Exemple 3 : (14alpha, 16alpha) 14,15-dihydro 20,21-dinoréburnaménin-14-ol**

On met en suspension 2,75 g du produit obtenu à l'exemple 1 dans 55 cm3 d'acide chlorhydrique 2N et chauffe une heure trente minutes à 50¤C. On ajoute à la solution obtenue 55 cm3 d'eau glacée et amène à un pH alcalin par addition de 10 cm3 d'ammoniaque 22 Bé et agite 15 minutes à température ambiante. On essore le précipité, lave à l'eau, sèche à 50¤C sous pression réduite et obtient 2,75 g de produit (mélange OH axial et équatorial). On chromatographie ce dernier sous pression, sur silice, élue par un mélange acétate d'éthyle - méthanol - ammoniaque (97-3-0,3). On obtient 1,70 g de produit (OH axial). F = 234¤C.
$[alpha]_D$ = + 150 ± 2 (c = 1% DMF).

Dichroïsme circulaire (dioxanne)

Max. : 230 nm $\Delta \epsilon$ = + 19
Max. : 290 nm $\Delta \epsilon$ = - 1,75

Spectre RMN 250 MHz (pyridine) ppm

Structure possible avec OH axial
Isomère OH équatorial non détecté.

>C Ⓗ OH : 6,26 (m)

**Exemple 4 : (3alpha, 14béta) 14,15-dihydro 20,21-dinoréburnaménin-14-ol.**

On opère comme à l'exemple 3 en partant de 13,3 g de produit obtenu à l'exemple 2 et obtient 7,7 g de produit (OH axial). F = 234¤C
$[alpha]_D$ = -152,5¤ ± 2,5¤ (c = 1% DMF)

Dichroïsme circulaire (dioxanne)

Max. : 228 nm $\Delta \epsilon$ = - 20
Max. : 290 nm $\Delta \epsilon$ = + 1,5
Spectre RMN (250 MHz) pyridine ppm
Structure possible avec OH axial
isomère avec OH équatorial non détecté.

>C Ⓗ OH : 6,23

**Exemple 5 : (3alpha) 20,21-dinoréburnaménine.**

On met en suspension 1,2 g du produit obtenu à l'exemple 2 dans 24 cm3 de toluène anhydre, ajoute 1 % d'acide paratoluènesulfonique et chauffe 4 heures au reflux. On amène à sec et reprend l'extrait sec par 50 cm3 d'acétate d'éthyle. On filtre l'insoluble et concentre à sec le filtrat. On chromatographie sur silice le résidu, élue par un mélange chlorure de méthylène - acétone (1-1) et obtient 0,610 g de produit attendu. F = 139¤C.
$[alpha]_D$ = + 445,5¤ ± 5¤ (c = 1% $CHCl_3$)

Dichroïsme circulaire (éthanol)

Max. : 257 nm $\Delta \epsilon$ = + 33
Infl.: 290 nm $\Delta \epsilon$ = + 3,5
Infl.: 299 nm $\Delta \epsilon$ = + 5,7
Infl.: 302 nm $\Delta \epsilon$ = + 6
Max. : 308 nm $\Delta \epsilon$ = + 7,8

Spectre RMN ($CDCl_3$) 250 MHz en ppm

Structure possible, fonction trans :

| | |
|---|---|
| éthyléniques : | 5,07 (dd, J = 2 et 7,5) |
| | 6,96 (dd, J = 3 et 7,5) |
| indole : | $H_5/H_6$ : 7,05 à 7,20 |
| | $H_4/H_7$ : 7,32 (d) et 7,46 (d) |
| autres protons : | 1,3 à 3,2 |

**Exemple 6 : (16alpha) 20,21-dinoréburnaménine.**

On opère comme à l'exemple 5 à partir de 1,2 g du produit obtenu à l'exemple 1 et obtient 0,640 g de produit attendu. F = 139¤C.
$[alpha]_D$ = -439¤ ± 5¤ (c = 1% $CHCl_3$)

Dichroïsme circulaire (éthanol)

Max. 257 nm $\Delta\epsilon$ = - 31
Infl. 290 nm $\Delta\epsilon$ = - 32
Infl. 300 nm $\Delta\epsilon$ = - 5,3
Max. 307 nm $\Delta\epsilon$ = - 7,4

Spectre RMN ($CDCl_3$) 250 MHz ppm

Structure possible, fonction trans

| | |
|---|---|
| éthyléniques : | 5,07 (dd, J = 2 et 7,5) |
| | 6,96 (dd, J = 3 et 7,5) |
| indole $H_5$ et $H_6$ : | 7,05 à 7,20 |
| $H_4$ et $H_7$ : | 7,32 (d) et 7,46 (d) |
| autres protons : | 1,3 à 3,2 |

**Exemple 7 : forme pharmaceutique.**

On a préparé des comprimés répondant à la formule suivante :

| | |
|---|---|
| - Produit de l'exemple 3 | 300 mg |
| - Excipient q.s. pour un comprimé | 350 mg. |

(Détail de l'excipient : talc, stéarate de magnésium, aérosil®).

**ETUDE PHARMACOLOGIOUE**

1) Affinité pour les récepteurs $alpha_2$ adrénergiques.

On homogénéise dans 90 ml de sucrose 0,32M, 10 cortex prélevés sur des cerveaux de rats mâles pesant 150 g en moyenne. Après centrifugation à 1000 g du mélange homogénéisé pendant 10 minutes à 0¤C, le surnageant est centrifugé à 30 000 g pendant 10 minutes à 0¤/4¤C. Le nouveau culot obtenu est mis en suspension dans 480 ml de tampon $NaKPO_4$ pH 7,4 50mM.

On fait ensuite incuber pendant 45 minutes à 25¤C, 2 ml de suspension en présence de $^3H$ rauwolscine à la concentration 0,15 nM :

i) seule,
ii) avec des concentrations croissantes du produit à tester ou,
iii) pour déterminer la fixation non spécifique,

avec de la phentolamine non radioactive à la concentration $10^{-5M.}$

Les suspensions incubées sont filtrées sur Whatman GF/C et les filtres sont lavés par trois fois 5 ml de tampon $NaKPO_4$ pH 7,4 à 0¤C.
La radioactivité des filtres est mesurée par scintillation liquide.

L'affinité du produit testé pour les récepteurs $alpha_2$ adrénergiquesest donnée relativement à la phentolamine comme produit de référence.

CD = concentration de phentolamine inhibant 50 % de la fixation spécifique de la $^3H$ rauwolscine ;

CX = concentration du produit à tester inhibant 50 % de la fixation spécifique de la $^3H$ rauwolscine.

L'affinité relative est donnée par la relation : $ARL = 100 \frac{CD}{CX}$

| Produits | ARL |
|---|---|
| Exemple 3 | 0,3 |
| Exemple 4 | 57 |
| Exemple 5 | 8 |
| Exemple 6 | 633 |

2) Test d'anoxie hypobare chez la souris.

Il consiste à mesurer sur une durée maximale de 3 minutes le temps de survie de souris placées dans une enceinte de 2 litres dans laquelle on réalise une dépression de 600 mmHg. On utilise des souris à jeûn depuis 6 heures. Les produits sont administrés par voie i.p. sous un volume de 0,2 ml/10 g 60 mn avant l'épreuve.

| Produit de l'exemple | temps de survie |
|---|---|
| 3 | + 37% |

3) Test de protection neuronale chez la souris.

Une lésion cérébrale est réalisée chez la souris par injection dans le striatum droit de 2 ug d'acide kaïnique sous un volume de 0,5 ul. Dans les heures qui suivent, le dommage se traduit par une libération sérique du marqueur neuronal (énolase gamma-gamma) proportionelle à l'étendue lésionnelle. Les produits sont injectés par voie intrapéritonéale 60 mn avant le neurotoxique et les prélèvements sanguins sont effectués 24 heures plus tard.

| Produit de l'exemple | énolase gamma-gamma |
|---|---|
| 3 | - 24 % |

4) Test d'activité antidépressive :

Les essais sont effectués sur des lots de 5 rats Sprague Dawley. Les animaux, naïfs, sont placés pendant 15 minutes dans un cylindre vertical en plexiglass (diamètre : 18 cm, hauteur : 40 cm) contenant de l'eau à 25¤C sur une hauteur de 15 cm (épreuve initiale de nage). Ils sont ensuite séchés pendant 15 minutes dans une enceinte chauffée à 32¤C, 24 heures plus tard, ils sont replacés dans le cylindre rempli d'eau et la durée totale des périodes d'immobilité est mesurée pendant 5 minutes.

Le composé est administré i.p. successivement 24,5 et 1/2 heure avant le test. La première administration a lieu immédiatement après l'épreuve initiale de nage, juste avant de replacer les animaux dans leur boîte d'élevage.

Les moyennes des groupes traités sont comparées à celles du groupe témoin par le test de Dunnett.

**Résultats :**

| Produit de l'exemple | $DA_{50}$ mg/kg |
|---|---|
| 3 | $\simeq$ 10 |
| 4 | $\simeq$ 5 |

**Effet anti-amnésiant dans un évitement passif.**

Des rats sont placés individuellement dans le compartiment éclairé d'une boîte à deux compartiments, l'autre étant obscure. Ils se réfugient spontanément dans le compartiment obscur et dès leur entrée, les rats reçoivent un choc électrique (1mA/5sec) par le plancher grillagé. Les animaux sont alors divisés en 3 groupes : le 1er groupe (témoin) ne subit pas d'autre manipulation. Dans le 2ème groupe, le choc électrique est immédiatement suivi de l'application d'un électrochoc amnésiant (60mA, 0,6ms, 0,6s) groupe témoin électrochoc). Le 3ème groupe est identique au 2ème, mais l'électrochoc est immédiatement suivi de l'administration du composé à tester (groupe traité). Vingt-quatre heures plus tard, les animaux sont replacés dans le compartiment éclairé de la boîte et on mesure le temps de latence d'entrée dans le compartiment sombre (jusqu'à 300 sec maxi). Chez les témoins, ce temps est voisin de 300 sec. Les témoins électrochoc pénètrent au contraire beaucoup plus rapidement dans le compartiment sombre (effet amnésiant). Les produits à effet anti-amnésiant augmentent la latence d'entrée et tendent à la ramener à une valeur comparable à celle des témoins sans électrochoc.

Les composés des exemples 3 et 4 réversent l'effet de l'électrochoc amnésiant aux doses respectives de 0,5 et 1 mg/kg i.p.

**Revendications**

**Revendications pour les Etats contractants suivants : AT, BE, CH, DE, FR, GB, IT, LI, LU, NL, SE**

**1.** Produits optiquement actifs des produits racémiques de formule (I) :

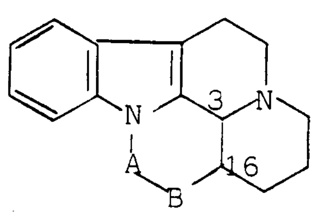

(I)

dans laquelle l'atome d'hydrogène en position 3 et l'atome d'hydrogène en position 16 sont trans et dans laquelle le groupement :

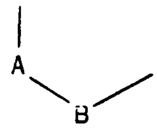

représente soit :

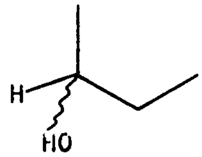

le radical hydroxy étant sous la forme alpha ou béta, soit :

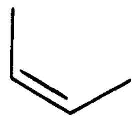

ainsi que leurs sels d'addition avec les acides minéraux ou organiques.

**2.** Produits optiquement actifs tels que définis à la revendication 1 des produits racémiques de formule (I) dans laquelle le groupement :

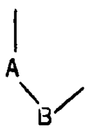

représente

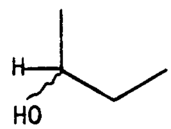

à savoir:

- le (14béta, 16alpha) 14,15-dihydro 20,21-dinoréburnamenin-14-ol,
- le (3alpha, 14béta) 14,15-dihydro 20,21-dinoréburnamenin-14-ol,
- le (14alpha, 16alpha) 14,15-dihydro 20,21-dinoréburnamenin-14-ol,
- le (3alpha, 14alpha) 14,15-dihydro 20,21-dinoréburnamenin-14-ol

ainsi que leurs sels d'addition avec les acides minéraux ou organiques.

**3.** Produits optiquement actifs tels que définis à la revendication 1 des produits racémiques de formule (I) dans laquelle le groupement :

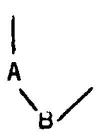

représente

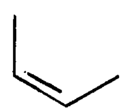

à savoir:

- la (16alpha) 20,21-dinoréburnaménine,
- la (3alpha) 20,21-dinoréburnaménine,

ainsi que leurs sels d'addition avec les acides minéraux ou organiques.

**4.** Procédé d'obtention des produits optiquement actifs tels que définis à la revendication 1, caractérisé en ce que :

a) l'on réduit soit le produit de formule (II) trans 3alpha, soit le produit de formule (II') trans 16alpha :

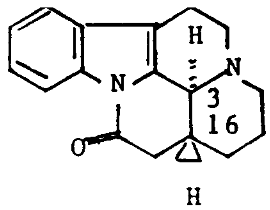

(II)

trans 3alpha

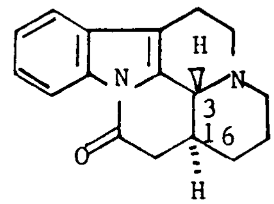

(II')

trans 16alpha

pour obtenir respectivement soit le produit de formule ($I_A$) trans 3alpha, soit le produit de formule ($I'_A$) trans 16alpha, produits de formules ($I_A$) et ($I'_A$) correspondant aux produits de formule (I) dans laquelle le groupement :

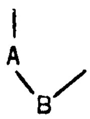

représente le groupement :

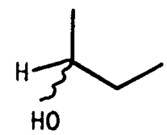

dans lequel le radical hydroxy est en position équatoriale :

b) on soumet ensuite, si désiré, soit le produit de formule ($I_A$) soit le produit de formule ($I'_A$) à l'action d'un acide pour obtenir soit le produit de formule ($I_{A1}$) trans 3alpha, soit le produit de formule ($I'_{A1}$) trans 16alpha, produits de formules ($I_{A1}$) et ($I'_{A1}$) correspondant aux produits de formule (I) dans laquelle le groupement :

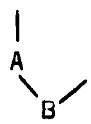

représente le groupement :

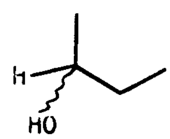

dans lequel le radical hydroxy est en position axiale :

c)on déshydrate ensuite, si désiré, soit l'un des deux produits de formules ($I_A$) ou ($I_{A1}$) soit l'un des deux produits de formules ($I'_A$) ou ($I'_{A1}$), pour obtenir soit le produit de formule ($I_C$) trans 3alpha, soit le produit de formule ($I'_C$) trans 16alpha, produits de formule ($I_C$) et ($I'_C$) correspondant aux produits de formule (I) dans laquelle le groupement :

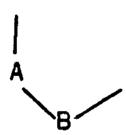

représente le groupement :

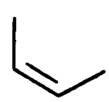

produits optiquement actifs que l'on soumet, si désiré, à l'action d'un acide minéral ou organique pour en former le sel.

**5.** A titre de médicaments, les produits optiquement actifs tels que définis à la revendication 1.

**6.** A titre de médicaments, les produits optiquement actifs dont les noms suivent :

- le (14alpha, 16alpha) 14,15-dihydro 20,21-dinoréburnamenin-14-ol,
- la (3alpha, 14béta) 14,15-dihydro 20,21-dinoréburnaménin-14-ol et leurs sels d'addition avec les acides.

**7.** Les compositions pharmaceutiques contenant à titre de principe actif, l'un au moins des médicaments tels que définis par les revendications 5 ou 6.

**Revendications pour l'Etat contractant suivant : ES**

**1.** Procédé pour préparer des produits optiquement actifs des produits racémiques de formule (I) :

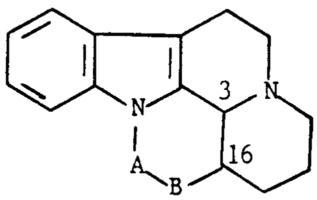

(I)

dans laquelle l'atome d'hydrogène en position 3 et l'atome d'hydrogène en position 16 sont trans et dans laquelle le groupement :

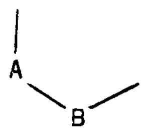

représente soit :

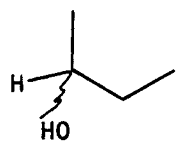

le radical hydroxy étant sous la forme alpha ou béta, soit :

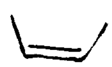

ainsi que leurs sels d'addition avec les acides minéraux ou organiques, caractérisé en ce que :

a) l'on réduit soit le produit de formule (II) trans 3alpha, soit le produit de formule (II') trans 16alpha :

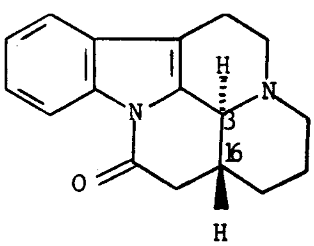

(II)

trans 3alpha

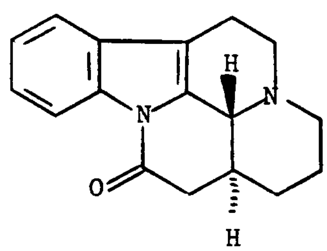

(II')

trans 16alpha

pour obtenir respectivement soit le produit de formule ($I_A$) trans 3alpha, soit le produit de formule ($I'_A$) trans 16alpha, produits de formules ($I_A$) et ($I'_A$) correspondant aux produits de formule (I) dans laquelle le groupement :

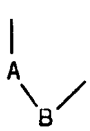

représente le groupement :

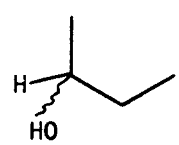

dans lequel le radical hydroxy est en position équatoriale :
b) on soumet ensuite, si désiré, soit le produit de formule ($I_A$) soit le produit de formule ($I'_A$) à l'action d'un acide pour obtenir soit le produit de formule ($I_{A1}$) trans 3alpha, soit le produit de formule ($I'_{A1}$) trans 16alpha, produits de formules ($I_{A1}$) et ($I'_{A1}$) correspondant aux produits de formule (I) dans laquelle le groupement :

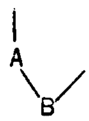

représente le groupement :

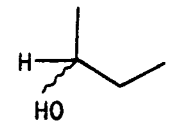

dans lequel le radical hydroxy est en position axiale :
c) on déshydrate ensuite, si désiré, soit l'un des deux produits de formules ($I_A$) ou ($I_{A1}$) soit l'un des deux produits de formules ($I'_A$) ou ($I'_{A1}$), pour obtenir soit le produit de formule ($I_C$) trans 3alpha, soit le produit de formule ($I'_C$) trans 16alpha, produits de formule ($I_C$) et ($I'_C$) correspondant aux produits de formule (I) dans laquelle le groupement :

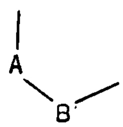

représente le groupement :

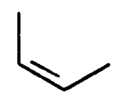

produits optiquement actifs que l'on soumet, si désiré, à l'action d'un acide minéral ou organique pour en former le sel.

**2.** Procédé selon la revendication 1, caractérisé en ce que l'on prépare l'un des produits dont les noms suivent :
- le (14béta, 16alpha) 14,15-dihydro 20,21-dinoréburnamenin-14-ol,
- le (3alpha, 14béta) 14,15-dihydro 20,21-dinoréburnamenin-14-ol,
- le (14alpha, 16alpha) 14,15-dihydro 20,21-dinoréburnamenin-14-ol,
- le (3alpha, 14alpha) 14,15-dihydro 20,21-dinoréburnamenin-14-ol

ainsi que leurs sels d'addition avec les acides minéraux ou organiques.

**3.** Procédé selon la revendication 1, caractérisé en ce que l'on prépare l'un des produits dont les noms suivent :
- la (16alpha) 20,21-dinoréburnaménine,
- la (3alpha) 20,21-dinoréburnaménine,

ainsi que leurs sels d'addition avec les acides minéraux ou organiques.

**Revendications pour l'Etat contractant suivant : GR**

**1.** Procédé pour préparer des produits optiquement actifs des produits racémiques de formule (I) :

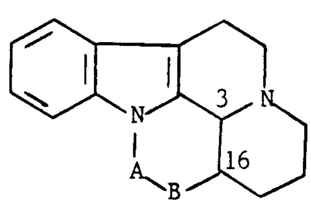

(I)

dans laquelle l'atome d'hydrogène en position 3 et l'atome d'hydrogène en position 16 sont trans et dans laquelle le groupement :

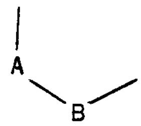

représente soit : 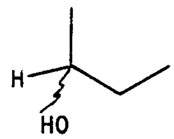

le radical hydroxy étant sous la forme alpha ou béta, soit :

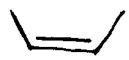

ainsi que leurs sels d'addition avec les acides minéraux ou organiques, caractérisé en ce que :

a) l'on réduit soit le produit de formule (II) trans 3alpha, soit le produit de formule (II') trans 16alpha :

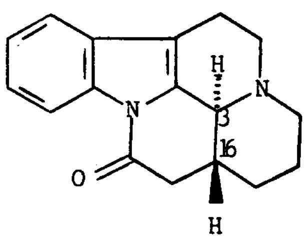

(II)

trans 3alpha

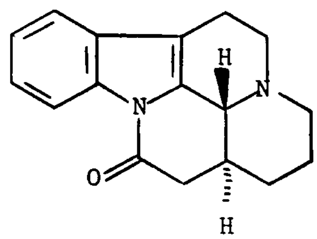

(II')

trans 16alpha

pour obtenir respectivement soit le produit de formule ($I_A$) trans 3alpha, soit le produit de formule ($I'_A$) trans 16alpha, produits de formules ($I_A$) et ($I'_A$) correspondant aux produits de formule (I) dans laquelle le groupement :

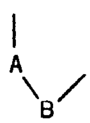

représente le groupement : 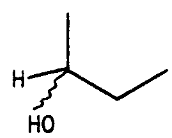

dans lequel le radical hydroxy est en position équatoriale :

b) on soumet ensuite, si désiré, soit le produit de formule ($I_A$) soit le produit de formule ($I'_A$) à l'action d'un acide pour obtenir soit le produit de formule ($I_{A1}$) trans 3alpha, soit le produit de formule ($I'_{A1}$) trans 16alpha, produits de formules ($I_{A1}$) et ($I'_{A1}$) correspondant aux produits de formule (I) dans laquelle le groupement :

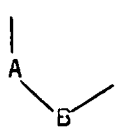

représente le groupement : 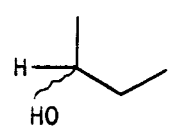

dans lequel le radical hydroxy est en position axiale :

c)on déshydrate ensuite, si désiré, soit l'un des deux produits de formules ($I_A$) ou ($I_{A1}$) soit l'un des deux produits de formules ($I'_A$) ou ($I'_{A1}$), pour obtenir soit le produit de formule ($I_C$) trans 3alpha, soit le produit de formule ($I_C$) trans 16alpha, produits de formule ($I_C$) et ($I'_C$) correspondant aux produits de formule (I) dans laquelle le groupement :

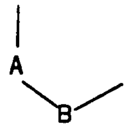 représente le groupement : 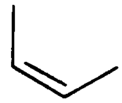

produits optiquement actifs que l'on soumet, si désiré, à l'action d'un acide minéral ou organique pour en former le sel.

**2.** Procédé selon la revendication 1, caractérisé en ce que l'on prépare l'un des produits dont les noms suivent :
- le (14béta, 16alpha) 14,15-dihydro 20,21-dinoréburnamenin-14-ol,
- le (3alpha, 14béta) 14,15-dihydro 20,21-dinoréburnamenin-14-ol,
- le (14alpha, 16alpha) 14,15-dihydro 20,21-dinoréburnamenin-14-ol,
- le (3alpha, 14alpha) 14,15-dihydro 20,21-dinoréburnamenin-14-ol

ainsi que leurs sels d'addition avec les acides minéraux ou organiques.

**3.** Procédé selon la revendication 1, caractérisé en ce que l'on prépare l'un des produits dont les noms suivent :
- la (16alpha) 20,21-dinoréburnaménine,
- la (3alpha) 20,21-dinoréburnaménine,

ainsi que leurs sels d'addition avec les acides minéraux ou organiques.

**4.** Procédé de préparation de compositions pharmaceutiques, caractérisées en ce que l'on met à titre de principe actif l'un au moins des produits de formule générale (I) telle que définie à la revendication 1 ou l'un au moins de leurs sels d'addition avec les acides minéraux ou organiques pharmaceutiquement acceptables sous une forme destinée à cet usage.

**5.** Procédé de préparation de compositions pharmaceutiques, caractérisées en ce que l'on met à titre de principe actif l'un au moins des produits de formule générale (I) dont les noms suivents :
- le (14alpha, 16alpha) 14,15-dihydro 20,21-dinoréburnamenin-14-ol,
- le (3alpha, 14béta) 14,15-dihydro 20,21-dinoréburnamenin-14-ol et leurs sels d'addition avec les acides, sous une forme destinée à cet usage.

## Claims

**Claims for the following Contracting States : AT, BE, CH, DE, FR, GB, IT, LI, LU, NL, SE**

**1.** The optically active products of the racemic products of formula (I):

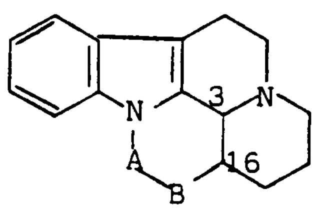

(I)

in which the hydrogen atom in position 3 and the hydrogen atom in position 16 are <u>trans</u> and in which the group:

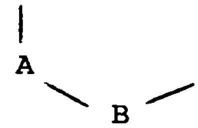

represents either:

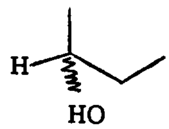

the hydroxy radical being in the alpha or beta form, or:

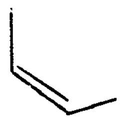

as well as their addition salts with mineral or organic acids.

**2.** The optically active products as defined in claim 1 of the racemic products of formula (I) in which the group:

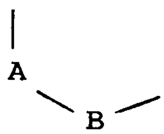

represents

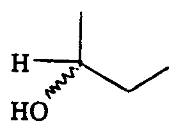

namely:

- (14beta, 16alpha) 14,15-dihydro 20,21-dinoreburnamenin-14-ol,
- (3alpha, 14beta) 14,15-dihydro 20,21-dinoreburnamenin-14-ol,
- (14alpha, 16alpha) 14,15-dihydro 20,21-dinoreburnamenin-14-ol,
- (3alpha, 14alpha) 14,15-dihydro 20,21-dinoreburnamenin-14-ol

as well as their addition salts with mineral or organic acids.

**3.** The optically active products as defined in claim 1 of the racemic products of formula (I) in which the group:

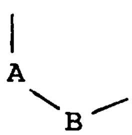

represents

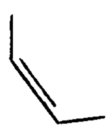

namely:

- (16alpha) 20,21-dinoreburnamenine,
- (3alpha) 20,21-dinoreburnamenine,

as well as their addition salts with mineral or organic acids.

**4.** Process for obtaining the optically active products as defined in claim 1, characterized in that:

a) either the product of formula (II) trans 3alpha, or the product of formula (II') trans 16alpha:

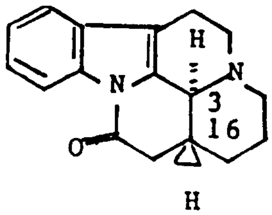 (II) 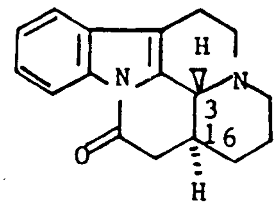 (II')

trans 3alpha

trans 16alpha

are reduced in order to obtain respectively either the product of formula ($I_A$) trans 3alpha, or the product of formula ($I'_A$) trans 16alpha, the products of formulae ($I_A$) and ($I'_A$) corresponding to the products of formula (I) in which the group:

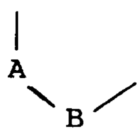 represents the group: 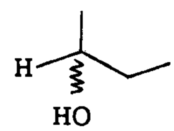

in which the hydroxy radical is in equatorial position:

b) then, if desired, either the product of formula ($I_A$) or the product of formula ($I'_A$) is subjected to the action of an acid in order to obtain either the product of formula ($I_{A1}$) trans 3alpha, or the product of formula ($I'_{A1}$) trans 16alpha, the products of formulae ($I_{A1}$) and ($I'_{A1}$) corresponding to the products of formula (I) in which the group:

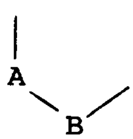 represents the group: 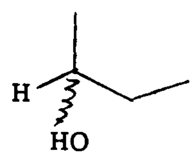

in which the hydroxy radical is in axial position:

c) then, if desired, either one of the two products of formulae ($I_A$) or ($I_{A1}$) or one of the two products of formulae ($I'_A$) or ($I'_{A1}$) is dehydrated in order to obtain either the product of formula ($I_C$) trans 3alpha, or the product of formula ($I'_C$) trans 16alpha, the products of formula ($I_C$) and ($I'_C$) corresponding to the products of formula (I) in which the group:

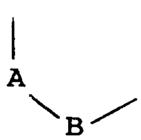 represents the group: 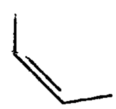

which optically active products are subjected, if desired, to the action of a mineral or organic acid in order to form the salt.

**5.** As medicaments, the optically active products as defined in claim 1.

**6.** As medicaments, the optically active products the names of which follow:

- (14alpha, 16alpha) 14,15-dihydro 20,21-dinoreburnamenin-14-ol,
- (3alpha, 14beta) 14,15-dihydro 20,21-dinoreburnamenin-14-ol and their addition salts with acids.

7. The pharmaceutical compositions containing at least one of the medicaments as defined by claims 5 or 6 as active ingredient.

**Claims for the following Contracting State : ES**

1. Process for preparing the optically active products of the racemic products of formula (I):

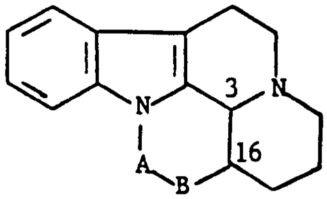

(I)

in which the hydrogen atom in position 3 and the hydrogen atom in position 16 are <u>trans</u> and in which the group:

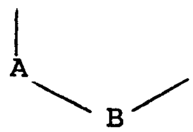

represents either: 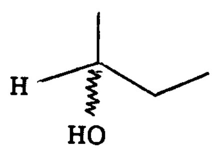

the hydroxy radical being in the alpha or beta form, or:

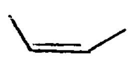

as well as their addition salts with mineral or organic acids, characterized in that:

a) either the product of formula (II) trans 3alpha, or the product of formula (II') trans 16alpha:

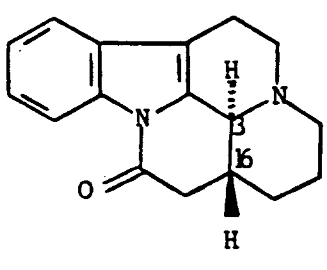

(II)

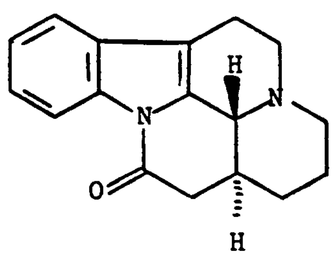

(II')

trans 3alpha

trans 16alpha

are reduced in order to obtain respectively either the product of formula ($I_A$) trans 3alpha, or the product of formula ($I'_A$) trans 16alpha, the products of formulae ($I_A$) and ($I'_A$) corresponding to the products of formula (I) in which the group:

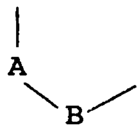 represents the group: 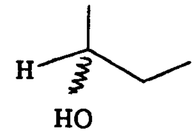

in which the hydroxy radical is in equatorial position:

b) then, if desired, either the product of formula ($I_A$) or the product of formula ($I'_A$) is subjected to the action of an acid in order to obtain either the product of formula ($I_{A1}$) trans 3alpha, or the product of formula ($I'_{A1}$) trans 16alpha, the products of formulae ($I_{A1}$) and ($I'_{A1}$) corresponding to the products of formula (I) in which the group:

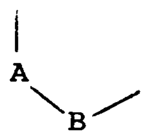 represents the group: 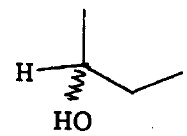

in which the hydroxy radical is in axial position:

c) then, if desired, either one of the two products of formulae ($I_A$) or ($I_{A1}$) or one of the two products of formulae ($I'_A$) or ($I'_{A1}$) is dehydrated in order to obtain either the product of formula ($I_C$) trans 3alpha, or the product of formula ($I'_C$) trans 16alpha, the products of formula ($I_C$) and ($I'_C$) corresponding to the products of formula (I) in which the group:

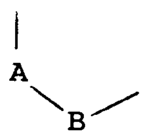 represents the group: 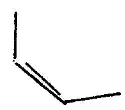

which optically active products are subjected, if desired, to the action of a mineral or organic acid in order to form the salt.

**2.** Process according to claim 1, characterized in that one of the products is prepared the names of which follow:
- (14beta, 16alpha) 14,15-dihydro 20,21-dinoreburnamenin-14-ol,
- (3alpha, 14beta) 14,15-dihydro 20,21-dinoreburnamenin-14-ol,
- (14alpha, 16alpha) 14,15-dihydro 20,21-dinoreburnamenin-14-ol,
- (3alpha, 14alpha) 14,15-dihydro 20,21-dinoreburnamenin-14-ol

as well as their addition salts with mineral or organic acids.

**3.** Process according to claim 1, characterized in that one of the products is prepared the names of which follow:
- (16alpha) 20,21-dinoreburnamenine,
- (3alpha) 20,21-dinoreburnamenine,

as well as their addition salts with mineral or organic acids.

**Claims for the following Contracting State : GR**

**1.** Process for preparing the optically active products of the racemic products of formula (I):

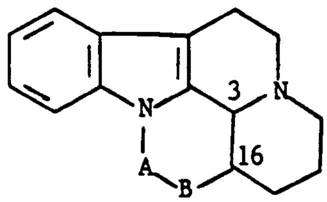

(I)

in which the hydrogen atom in position 3 and the hydrogen atom in position 16 are <u>trans</u> and in which the group:

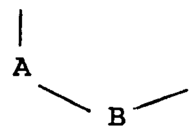

represents either: 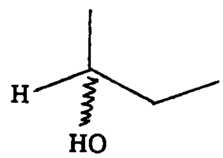

the hydroxy radical being in the alpha or beta form, or:

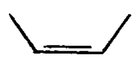

as well as their addition salts with mineral or organic acids, characterized in that:

a) either the product of formula (II) trans 3alpha, or the product of formula (II') trans 16alpha:

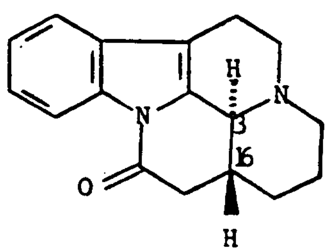

(II)

trans 3alpha

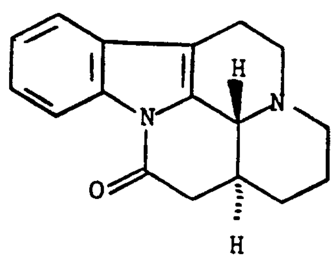

(II')

trans 16alpha

are reduced in order to obtain respectively either the product of formula ($I_A$) trans 3alpha, or the product of formula ($I'_A$) trans 16alpha, the products of formulae ($I_A$) and ($I'_A$) corresponding to the products of formula (I) in which the group:

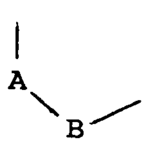

represents the group: 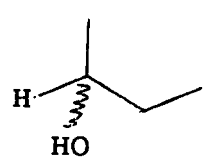

in which the hydroxy radical is in equatorial position:
b) then, if desired, either the product of formula ($I_A$) or the product of formula ($I'_A$) is subjected to the action of an acid in order to obtain either the product of formula ($I_{A1}$) trans 3alpha, or the product of formula ($I'_{A1}$) trans 16alpha, the products of formulae ($I_{A1}$) and ($I'_{A1}$) corresponding to the products of formula (I) in which the group:

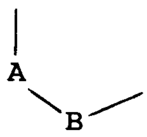

represents the group:

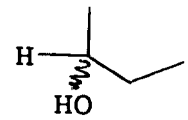

in which the hydroxy radical is in axial position:
c) then, if desired, either one of the two products of formulae ($I_A$) or ($I_{A1}$) or one of the two products of formulae ($I'_A$) or ($I'_{A1}$) is dehydrated in order to obtain either the product of formula ($I_C$) trans 3alpha, or the product of formula ($I'_C$) trans 16alpha, the products of formula ($I_C$) and ($I'_C$) corresponding to the products of formula (I) in which the group:

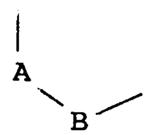

represents the group:

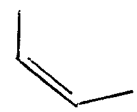

which optically active products are subjected, if desired, to the action of a mineral or organic acid in order to form the salt.

**2.** Process according to claim 1, characterized in that one of the products is prepared the names of which follow:
- (14beta, 16alpha) 14,15-dihydro 20,21-dinoreburnamenin-14-ol,
- (3alpha, 14beta) 14,15-dihydro 20,21-dinoreburnamenin-14-ol,
- (14alpha, 16alpha) 14,15-dihydro 20,21-dinoreburnamenin-14-ol,
- (3alpha, 14alpha) 14,15-dihydro 20,21-dinoreburnamenin-14-ol

as well as their addition salts with mineral or organic acids.

**3.** Process according to claim 1, characterized in that one of the products is prepared the names of which follow:
- (16alpha) 20,21-dinoreburnamenine,
- (3alpha) 20,21-dinoreburnamenine,

as well as their addition salts with mineral or organic acids.

**4.** Preparation process for pharmaceutical compositions, characterized in that at least one of the products of general formula (I) as defined in claim 1 or at least one of their addition salts with pharmaceutically acceptable mineral or organic acids is used as active ingredient in a form intended for this use.

**5.** Preparation process for pharmaceutical compositions, characterized in that at least one of the products of general formula (I) is used as active ingredient the names of which follow:
- (14alpha, 16alpha) 14,15-dihydro 20,21-dinoreburnamenin-14-ol,
- (3alpha, 14alpha) 14,15-dihydro 20,21-dinoreburnamenin-14-ol and their addition salts with acids, in a form intended for this use.

**Patentansprüche**
**Patentansprüche für folgende Vertragsstaaten : AT, BE, CH, DE, FR, GB, IT, LI, LU, NL, SE**

**1.** Optisch aktive Produkte der racemischen Produkte der Formel (I)

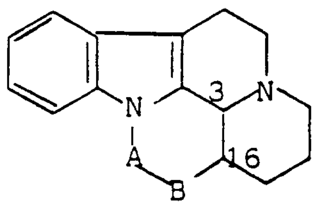

(I)

worin das Wasserstoffatom in 3-Stellung und das Wasserstoffatom in 16-Stellung trans-ständig sind, und worin die Gruppe

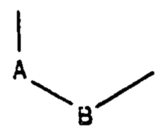

entweder für

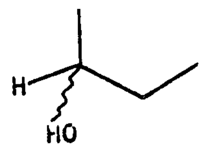

steht, wobei die Hydroxygruppe in der α- oder β-Form vorliegt, oder für

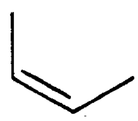

steht, sowie deren Additionssalze mit Mineral- oder organischen Säuren.

**2.** Optisch aktive Produkte, wie in Anspruch 1 definiert, der racemischen Produkte der Formel (I), worin die Gruppe

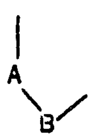

für

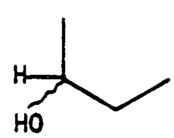

steht, nämlich:
- (14β,16α)-14,15-Dihydro-20,21-dinoreburnamenin-14-ol,
- (3α,14β)-14,15-Diyhdro-20,21-dinoreburnamenin-14-ol,
- (14α,16α)-14,15-Dihydro-20,21-dinoreburnamenin-14-ol,
- (3α,14α)-14,15-Dihydro-20,21-dinoreburnamenin-14-ol,

sowie deren Additionssalze mit Mineral- oder organischen Säuren.

**3.** Optisch aktive Produkte, wie in Anspruch 1 definiert, der racemischen Produkte der Formel (I), worin die Gruppe

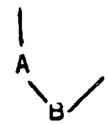

für

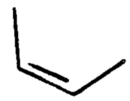

steht, nämlich:

- (16α)-20,21-Dinoreburnamenin,
- (3α)-20,21-Dinoreburnamenin,

sowie deren Additionssalze mit Mineral- oder organischen Säuren.

**4.** Verfahren zur Herstellung der optisch aktiven Produkte, wie in Anspruch 1 definiert, dadurch gekennzeichnet, daß man

a) entweder das trans-3α-Produkt der Formel (II) oder das trans-16α-Produkt der Formel (II')

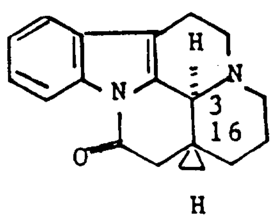

(II)

trans-3α

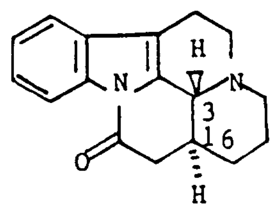

(II')

trans-16α

reduziert, um entweder zu dem trans-3α-Produkt der Formel ($I_A$) oder dem trans-16α-Produkt der Formel ($I'_A$) zu gelangen, wobei die Produkte der Formeln ($I_A$) und ($I'_A$) den Produkten der Formel (I) entsprechen, worin die Gruppe

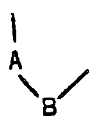

für die Gruppe

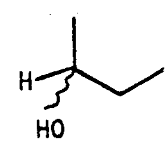

in welcher der Hydroxyrest in äquatorialer Position vorliegt, steht,

b) hierauf gewünschtenfalls entweder das Produkt der Formel ($I_A$) oder das Produkt der Formel ($I'_A$) der Einwirkung einer Säure unterzieht, um entweder zu dem trans-3α-Produkt der Formel ($I_{A1}$) oder zu dem trans-16α-Produkt der Formel ($I'_{A1}$) zu gelangen, wobei die Produkte der Formeln ($I_{A1}$) und ($I'_{A1}$) den Produkten der Formel (I) entsprechen, worin die Gruppe

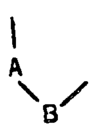

für die Gruppe

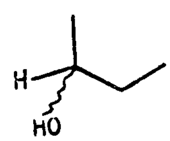

in der die Hydroxygruppe in axialer Position vorliegt, steht,

c) hierauf gewünschtenfalls entweder eines der beiden Produkte der Formeln ($I_A$) oder ($I_{A1}$) oder eines der beiden Produkte der Formeln ($I'_A$) oder ($I'_{A1}$) dehydratisiert, um entweder zu dem trans-3α-Produkt der Formel ($I_C$) oder dem trans-16α-Produkt der Formel ($I'_C$) zu gelangen, wobei die Produkte der Formeln ($I_C$) und ($I'_C$) den Produkten der Formel (I) entsprechen, worin die Gruppe

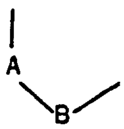 für die Gruppe 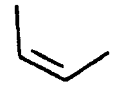

steht, und gewünschtenfalls die optisch aktiven Produkte der Einwirkung einer Mineral- oder organischen Säure unterzieht, um hieraus das Salz zu bilden.

**5.** Als Arzneimittel die optisch aktiven Produkte, wie in Anspruch 1 definiert.

**6.** Als Arzneimittel die optisch aktiven Produkte mit den folgenden Bezeichnungen
- (14α,16α)-14,15-Dihydro-20,21-dinoreburnamenin-14-ol,
- (3α,14β)-14,15-Dihydro-20,21-dinoreburnamenin-14-ol,

und deren Additionssalze mit Säuren.

**7.** Pharmazeutische Zusammensetzungen, enthaltend als Wirkstoff zumindest eines der Arzneimittel, wie in den Ansprüchen 5 oder 6 definiert.

**Patentansprüche für folgenden Vertragsstaat : ES**

**1.** Verfahren zur Herstellung der optisch aktiven Produkte der racemischen Produkte der Formel (I)

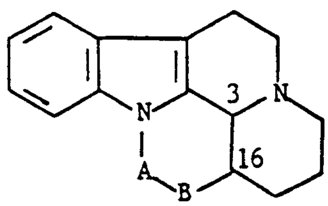

(I)

worin das Wasserstoffatom in 3-Stellung und das Wasserstoffatom in 16-Stellung trans-ständig sind, und worin die Gruppe

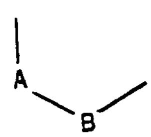

für

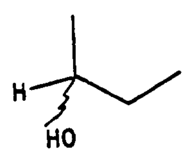

worin der Hydroxyrest in der α- oder β-Form vorliegt, oder

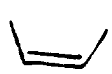

steht, sowie von deren Additionssalzen mit Mineral- oder organischen Säuren, dadurch gekennzeichnet, daß man:

a) entweder das trans-3α-Produkt der Formel (II) oder das trans-16α-Produkt der Formel (II')

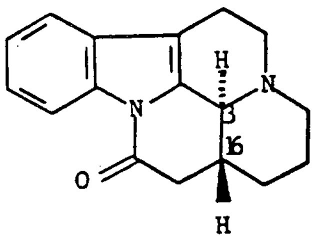

(II)

trans-3α

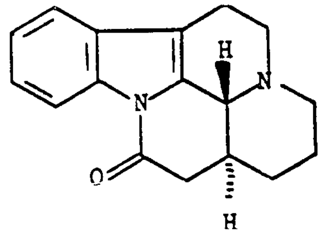

(II')

trans-16α

reduziert, um entweder zu dem trans-3α-Produkt der Formel ($I_A$) oder dem trans-16α-Produkt der Formel ($I'_A$) zu gelangen, wobei die Produkte der Formeln ($I_A$) und ($I'_A$) den Produkten der Formel (I) entsprechen, worin die Gruppe

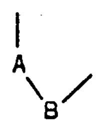

für die Gruppe

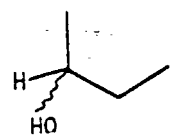

worin der Hydroxyrest sich in äquatorialer Position befindet, steht,

b) hierauf gewünschtenfalls entweder das Produkt der Formel ($I_A$) oder das Produkt der Formel ($I'_A$) der Einwirkung einer Säure unterzieht, um entweder zu dem trans-3α-Produkt der Formel ($I_{A1}$) oder zu dem trans-16α-Produkt der Formel ($I'_{A1}$) zu gelangen, wobei die Produkte der Formeln ($I_{A1}$) und ($I'_{A1}$) den Produkten der Formel (I) entsprechen, worin die Gruppe

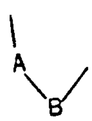

für die Gruppe

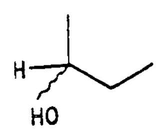

worin die Hydroxygruppe sich in axialer Position befindet, steht,

c) hierauf gewünschtenfalls entweder eines der beiden Produkte der Formeln ($I_A$) oder ($I_{A1}$) oder eines der beiden Produkte der Formeln ($I'_A$) oder ($I'_{A1}$) dehydratisiert, um entweder zu dem trans-3α-Produkt der Formel ($I_C$) oder zu dem trans-16α-Produkt der Formel ($I'_C$) zu gelangen, wobei die Produkte der Formel ($I_C$) und ($I'_C$) den Produkten der Formel (I) entsprechen, worin die Gruppe

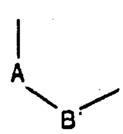

für die Gruppe

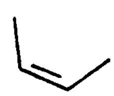

steht, und gewünschtenfalls die optisch aktiven Produkte der Einwirkung einer Mineral- oder organischen Säure unterzieht, um hieraus das Salz zu bilden.

**2.** Verfahren gemäß Anspruch 1, dadurch gekennzeichnet, daß man eines der Produkte mit den folgenden Bezeichnungen

- (14β,16α)-14,15-Dihydro-20,21-dinoreburnamenin-14-ol,
- (3α,14β)-14,15-Diyhdro-20,21-dinoreburnamenin-14-ol,
- (14α,16α)-14,15-Dihydro-20,21-dinoreburnamenin-14-ol,

- (3α,14α)-14,15-Dihydro-20,21-dinoreburnamenin-14-ol,

sowie deren Additionssalze mit Mineral- oder organischen Säuren herstellt.

**3.** Verfahren gemäß Anspruch 1, dadurch gekennzeichnet, daß man eines der Produkte mit den folgenden Bezeichnungen
- (16α)-20,21-Dinoreburnamenin,
- (3α)-20,21-Dinoreburnamenin,

sowie deren Additionssalze mit Mineral- oder organischen Säuren herstellt.

**Patentansprüche für folgenden Vertragsstaat : GR**

**1.** Verfahren zur Herstellung der optisch aktiven Produkte der racemischen Produkte der Formel (I)

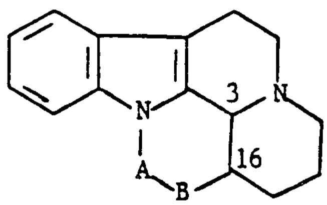

(I)

worin das Wasserstoffatom in 3-Stellung und das Wasserstoffatom in 16-Stellung trans-ständig sind, und worin die Gruppe

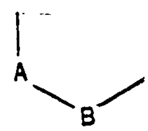

für

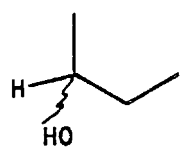

worin der Hydroxyrest in der α- oder β-Form vorliegt, oder

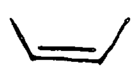

steht, sowie von deren Additionssalzen mit Mineral- oder organischen Säuren, dadurch gekennzeichnet, daß man:

a) entweder das trans-3α-Produkt der Formel (II) oder das trans-16α-Produkt der Formel (II')

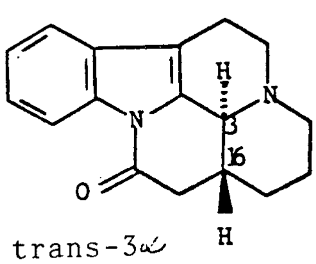

(II)

trans-3α

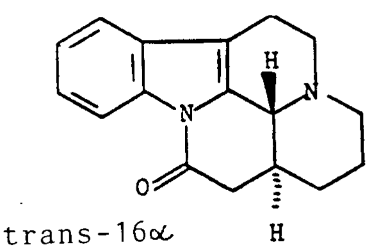

(II')

trans-16α

reduziert, um entweder zu dem trans-3α-Produkt der Formel ($I_A$) oder dem trans-16α-Produkt der Formel ($I'_A$) zu gelangen, wobei die Produkte der Formeln ($I_A$) und ($I'_A$) den Produkten der Formel (I) entsprechen, worin die Gruppe

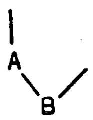 für die Gruppe 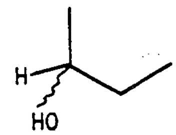

worin der Hydroxyrest sich in äquatorialer Position befindet, steht,
b) hierauf gewünschtenfalls entweder das Produkt der Formel ($I_A$) oder das Produkt der Formel ($I'_A$) der Einwirkung einer Säure unterzieht, um entweder zu dem trans-3α-Produkt der Formel ($I_{A1}$) oder zu dem trans-16α-Produkt der Formel ($I'_{A1}$) zu gelangen, wobei die Produkte der Formeln ($I_{A1}$) und ($I'_{A1}$) den Produkten der Formel (I) entsprechen, worin die Gruppe

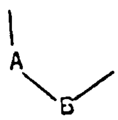 für die Gruppe 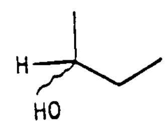

worin die Hydroxygruppe sich in axialer Position befindet, steht,
c) hierauf gewünschtenfalls entweder eines der beiden Produkte der Formeln ($I_A$) oder ($I_{A1}$) oder eines der beiden Produkte der Formeln ($I'_A$) oder ($I'_{A1}$) dehydratisiert, um entweder zu dem trans-3α-Produkt der Formel ($I_C$) oder zu dem trans-16α-Produkt der Formel ($I'_C$) zu gelangen, wobei die Produkte der Formel ($I_C$) und ($I'_C$) den Produkten der Formel (I) entsprechen, worin die Gruppe

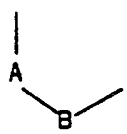 für die Gruppe 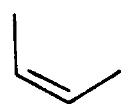

steht, und gewünschtenfalls die optisch aktiven Produkte der Einwirkung einer Mineral- oder organischen Säure unterzieht, um hieraus das Salz zu bilden.

**2.** Verfahren gemäß Anspruch 1, dadurch gekennzeichnet, daß man eines der Produkte mit den folgenden Bezeichnungen

- (14β,16α)-14,15-Dihydro-20,21-dinoreburnamenin-14-ol,
- (3α,14β)-14,15-Diyhdro-20,21-dinoreburnamenin-14-ol,
- (14α,16α)-14,15-Dihydro-20,21-dinoreburnamenin-14-ol,
- (3α,14α)-14,15-Dihydro-20,21-dinoreburnamenin-14-ol,

sowie deren Additionssalze mit Mineral- oder organischen Säuren herstellt.

**3.** Verfahren gemäß Anspruch 1, dadurch gekennzeichnet, daß man eines der Produkte mit den folgenden Bezeichnungen

- (16α)-20,21-Dinoreburnamenin,
- (3α)-20,21-Dinoreburnamenin,

sowie deren Additionssalze mit Mineral- oder organischen Säuren herstellt.

**4.** Verfahren zur Herstellung von pharmazeutischen Zusammensetzungen, dadurch gekennzeichnet, daß man als Wirkstoff zumindest eines der Produkte der allgemeinen Formel (I), wie in Anspruch 1 definiert, oder zumindest eines ihrer pharmazeutisch verträglichen Additionssalze mit Mineral- oder organischen Säuren in eine für diese Verwendung bestimmte Form überführt.

5. Verfahren zur Herstellung von pharmazeutischen Zusammensetzungen, dadurch gekennzeichnet, daß man als Wirkstoff zumindest eines der Produkte der allgemeinen Formel (I) mit den folgenden Bezeichnungen
   - (14α,16α)-14,15-Dihydro-20,21-dinoreburnamenin-14-ol,
   - (3α,14β)-14,15-Dihydro-20,21-dinoreburnamenin-14-ol, und deren Additionssalze mit Säuren in eine für diese Verwendung bestimmte Form überführt.